# EUROPEAN PATENT APPLICATION

(11) **EP 1 932 923 A1**
(43) Date of publication of application: **18.06.2008**
(21) Application number: 06797683.7
(22) Date of filing: 08.09.2006
(51) Int. Cl.: C12Q 1/68, C12N 15/09

(54) **METHOD FOR DETERMINATION OF EFFECTIVE DOSE OF METHOTREXATE**

(30) Priority: 12.09.2005 JP 2005263973
(71) Applicant: StaGen Co., Ltd., Taito-ku Tokyo 111-0051 (JP)
(72) Inventor: KAMATANI, Naoyuki, Tokyo 1620054 (JP); TANIGUCHI, Atsuo, Tokyo 1620054 (JP)
(74) Representative: Thornton, Neil
(86) International application number: PCT/JP2006/317828
(87) International publication number: WO 2007/032264

(57) **Abstract**

Problems: Provided are a test method of determining an effective dose of methotrexate in each patient and a method for treatment of rheumatoid arthritis.

Means to Solve the Problems: There is obtained a test method of an A1298C polymorphism in MTHFR gene in a patient suffering from rheumatoid arthritis, the method being used for the determination of the effective dose of methotrexate in each patient, whereby a method for treatment of rheumatoid arthritis in each patient is also obtained.

[Sequence Listing]
SEQUENCE LISTING
<110> StaGen.Co.Ltd.
<120> The estimation method of correct dose for methotrexate
<130> P-TWMU06
<140>
<141>
<150> JP 2005-263973
<151> 2005-9-12
<160> 2
<170> PatentIn Ver. 2.1
<210> 1
<211> 21
<212> DNA
<213> Artificial Sequence
<220>
<223> Description of Artificial Sequence:Primer

<400> 1

gggaggagct gaccagtgca g

21

<210> 2

<211> 19

<212> DNA

<213> Artificial Sequence

<220>

<223> Description of Artificial Sequence:Primer

<400> 2

ggggtcaggc caggggcag

19

SEQUENCE LISTING

<110> StaGen.Co.Ltd.

<120> The estimation method of correct dose for methotrexate

<130> P-TWMU06

<140>
<141>

<150> JP 2005-263973
<151> 2005-9-12

<160> 2

<170> PatentIn Ver. 2.1

<210> 1
<211> 21
<212> DNA
<213> Artificial Sequence

<220>
<223> Description of Artificial Sequence:Primer

<400> 1
gggaggagct gaccagtgca g                                                21

<210> 2
<211> 19
<212> DNA
<213> Artificial Sequence

<220>
<223> Description of Artificial Sequence:Primer

<400> 2
ggggtcaggc caggggcag                                                   19

## Description

### TECHNICAL FIELD

The present invention relates to a method of determining an effective dose of methotrexate, which is an antirheumatic, in a patient suffering from rheumatoid arthritis. In addition, the present invention relates to a method for treatment of rheumatoid arthritis, in which a dose of methotrexate for each individual is determined by using the method of determining the dose of methotrexate.

### BACKGROUND ART

Methotrexate is one of effective drugs which are administered most commonly as a therapeutic agent for rheumatoid arthritis (see, for example, Non-Patent Document 1). The methotrexate is known as being capable of not only temporarily inhibiting pain, but also of inhibiting joint destruction caused by the rheumatoid arthritis to improve prognosis of the rheumatoid arthritis (see, for example, Non-Patent Document 2).

However, a necessary dose of the methotrexate for inhibiting the rheumatoid arthritis remarkably varies depending on each individual. For example, the administration of the methotrexate in 2 mg/week produces a sufficient effect to some patients, but the administration of the methotrexate in 15 mg/week does not produce the sufficient effect to other patients. It is thought to be necessary to increase the dose of the methotrexate as soon as possible, with the maximum dose of 15 mg/week, for the patients who cannot gain the sufficient effect, while considering adverse side effects and the like (see, for example, Non-Patent Document 1).
Therefore, the dose of the methotrexate for a patient who has been treated for a sufficient period of time since the start of treatment reflects a personal effective dose of the methotrexate for the patient. A patient who is likely to get an effect of the methotrexate tends to finally have a low dose of the methotrexate, and a patient who is not likely to get the effect thereof tends to finally have a high dose of the methotrexate. However, it was difficult to know the effective dose of the methotrexate for each patient beforehand.

It is recently suggested that there is a relationship between the effective dose of the methotrexate for each patient and a gene of methylenetetrahydrofolate reductase or 5,10-methylenetetrahydrofolate reductase, abbreviated as MTHFR, EC 1.5.1.20.
In other words, it is reported that there is a relationship between A1298C polymorphism present in the seventh exon in the MTHFR gene (see, for example, Non-Patent Document 4) and a dose of methotrexate for rheumatoid arthritis (see, for example, Non-Patent Document 3). The report shows that a cohort study was conducted, in which a final dose of the methotrexate was sought against 99 patients who, suffering from the rheumatoid arthritis, had been administered with the methotrexate for at least 3 months after the start of administration. In addition, they studied the relationships between genotypes of A1298C in the MTHFR gene in those patients and the final dose of the methotrexate. The report shows that a ratio in which more than 5 mg of the methotrexate was administered was higher in 68 individuals each having an A/A genotype (Primary group) than 31 individuals each having any of the genotypes excluding the A/A genotype (A/C and C/C, Second group), and a proportion ratio of the former and the latter was 1.78. In other words, the report shows that a probability that patients suffering from the rheumatoid arthritis each having the A/A genotype are finally administered with the methotrexate in more than 5 mg/week was 1.78 times that of the patients having other genotypes.

However, though the Non-Patent Document 3 reports that the patients each having the A/A genotype were likely to be administered with high doses of the methotrexate, no investigation was done a relationship between each of the genotypes of the A/C or the C/C and the dose of the methotrexate. Therefore, in the treatment of the rheumatoid arthritis, there has been no high precision determination method capable of grasping the effective dose of the methotrexate for each patient in advance to determine the dose.
[Non-Patent Document 1] American College of Rheumatology Subcommittee on Rheumatoid Arthritis Guidelines. Guidelines for the management of rheumatoid arthritis: 2002 Update. Arthritis Rheum. 2002;46(2):328-46.
[Non-Patent Document 2] Choi HK, Hernan MA, Seeger JD, Robins JM, Wolfe F. Methotrexate and mortality in patients with rheumatoid arthritis: a prospective study. Lancet. 2002; 359(9313):1173-7
[Non-Patent Document 3] Urano W, Taniguchi A, Yamanaka H, Tanaka E, Nakajima H, Matsuda Y, Akama H, Kitamura Y, Kamatani N. Polymorphisms in the methylenetetrahydrofolate reductase gene were associated with both the efficacy and the toxicity of methotrexate used for the treatment of rheumatoid arthritis, as evidenced by single locus and haplotype analyses.Pharmacogenetics. 2002;12(3):183-90.
[Non-Patent Document 4] van der Put NM, Gabreels F, Stevens EM, Smeitink JA, Trijbels FJ, Eskes TK, van den Heuvel LP, Blom HJ. A second common mutation in the methylenetetrahydrofolate reductase gene: an additional risk factor for neural-tube defects Am J Hum Genet. 1998;62(5):1044-51.

### DISCLOSURE OF THE INVENTION

### PROBLEM TO BE SOLVED BY THE INVENTION

It is an object of the present invention to provide a test method for grasping an effective dose of methotrexate for each patient in treatment of rheumatoid arthritis. Another object of the present invention is to provide a method for treatment of rheumatoid arthritis in which a dose of methotrexate for each individual is determined.

### MEANS FOR SOLVING THE PROBLEM

The inventors of the present invention intensively studied to solve the above-mentioned problems. As a result, the inventors found that the effective dose of methotrexate for each patient may be determined by examining A1298C polymorphism in the MTHFR gene in the each individual patient suffering from the rheumatoid arthritis. In particular, the inventors examined which one of three genotypes the A1298C polymorphism belongs to and found that it could be determined that a probability of being an high effective dose of the methotrexate, is lower in a patient having the C/C genotype (C allele only), medium in a patient having the A/C genotype, and higher in a patient having the A/A genotype (A allele only). Thus, the method of determining the dose of the methotrexate for each patient in the treatment of the rheumatoid arthritis was obtained to complete the present invention.

Namely, the present invention relates to the following items (1) to (4).
(1) A test method of determining an effective dose of methotrexate in a patient suffering from rheumatoid arthritis, including examining which one of three types, A/A, A/C, or C/C, an A1298C polymorphism in an MTHFR gene in the patient belongs to.
(2) A method of determining an effective dose of methotrexate for each individual patient by examining which one of three types, A/A, A/C, or C/C, an A1298C polymorphism in an MTHFR gene in the patient belongs to.
(3) A method according to the above item (2), including judging that a possibility that an effective dose is high, is low in a patient having the C/C genotype with a C allele only, medium in a patient having the A/C genotype, and high in a patient having the A/A genotype with an A allele only.
(4) A method for treatment of rheumatoid arthritis for determining a dose of methotrexate for each individual using the method according to the above item (3) or (4).

### EFFECTS OF THE INVENTION

Use of the determination method of the present invention for a laboratory test and the like enables the determination of the dose for each patient at start of the administration in each treatment and the determination on how much the dose for each patient should be increased in the case of the insufficient effect. This method enables prevention of excessive administration of the methotrexate, reduction of burden on a patient body, and reduction of medical expenses. In addition, this method can lead each patient suffering from the rheumatoid arthritis to treatment with the effective dose earlier than a conventional method and enables prevention of progress of the rheumatoid arthritis.

### BRIEF DESCRIPTION OF THE DRAWINGS

[Figure 1] is a diagram showing a relationship between genotypes of A1298C polymorphism in an MTHFR gene and ratios of individuals to whom a final dose of methotrexate (more than 6 mg/week) was administered 12 months after start of an administration (Example).
[Figure 2] is a diagram showing a relationship between genotypes of A1298C polymorphism in an MTHFR gene and ratios of individuals to whom a final dose of the methotrexate (more than 6 mg/week) was administered 12 months after start of an administration (Comparative Example).

### BEST MODE FOR CARRYING OUT THE INVENTION

The term "a patient suffering from rheumatoid arthritis" according to the present invention refers to a patient who was diagnosed by a doctor as a patient with rheumatoid arthritis and using methotrexate as a therapeutic agent. Any patient suffering from the rheumatoid arthritis such as a patient suffering from the rheumatoid arthritis is included.

The term "the effective dose of the methotrexate" according to the present invention refers to a dose in which the methotrexate shows an effect in an individual patient suffering from the rheumatoid arthritis. An effect of the methotrexate is judged by a fact that symptoms such as joint pain, swelling, and the like in the individual patient are relieved by the administration of the methotrexate.
Two mg/week of the methotrexate is administered in common treatment. However, in the case of a patient who cannot attain a sufficient effect, 6 mg/week or more is administered. Considering the above fact, as the effective dose of the methotrexate in the present invention, a dose of less than 6 mg/week was defined as a low dose, and the dose of 6 mg/week or more was defined as a high dose.

Note that, in general, a 2 mg tablet and 2.5 mg tablet are used as a tablet for the methotrexate. Since the 2 mg tablets were used in Example of the present invention, a criterion to determine the low dose and high dose was set at 6 mg. On the other hand, 5 mg was set as a criterion in the case of using the 2.5 mg tablets in Non-Patent Document 3. The fact indicates that a criterion varies depending on a tablet used.

The phrase "test method for determining an effective dose of methotrexate" according to the present invention refers to a method which is used for examining whether the effective dose of the methotrexate for an individual patient belongs to the low dose or high dose.
In the present invention, by examining which one of three types, A/A, A/C, or C/C, the A1298C polymorphism in the MTHFR gene in an individual patient belongs to, a possibility that the individual patient belongs to which doses, a low dose or high dose, depending on a genotype the patient has can be judged. It is judged that a possibility that the effective dose of the present invention is high, is high in a patient having the C/C genotype (C allele only) in A1298C polymorphism in the MTHFR gene, medium in a patient having the A/C genotype, and high in a patient having the A/A genotype (A allele only).
Since the genotype of an individual is permanent, one examination is effective life-time long for an individual who was examined. Therefore, regarding a patient whose genotype was examined by the method of the present invention, an effective dose of the methotrexate can be permanently determined based on information obtained from the examination of the genotype.

Although an action mechanism of the methotrexate is thought to be caused by reduction of folic acid, the following Table 1 abstracted from Non-Patent Document 4 shows that an enzyme activity of MTHFR declines in the order of A/A > A/C > C/C, but there is no difference in folic acid concentrations in serum.

**[Table 1]**

| Comparison of an enzyme activity in individuals each having a genotype of A1298C in the MTHFR (in case w here A/A is 100%) and folic acid concentrations in serum (abstracted from Non-Patent Document 4) | | | |
|---|---|---|---|
| Genotype | A/A | A/C | C/C |
| Enzyme activity | 100% | 83.2% | 61.1% |
| Folic acid concentration in serum (nmol/L) | 12.6±5.5 | 12.5±4.5 | 13.5±4.2 |

As shown above, reduction of an enzyme activity for each genotype is indicated, but reduction of folic acid concentration is not shown. Therefore, it cannot be regarded at all that there is a relationship between A1298C gene polymorphism and an effective dose of the methotrexate. For example, it is indicated that the enzyme activity of MTHFR varies depending on a genotype of C677T polymorphism in the MTHFR much more than that of the A1298C, and the C677T polymorphism is also associated with the difference in the folic acid concentrations (Non-Patent Document 4). However, there is no relationship between genotypes of the C677T and effective doses of the methotrexate (Table 5 of Non-Patent Document 3). As described above, there is a large gap in the relationship between an enzyme activity, concentrations of a substance, and efficacy of a drug. It is a common sense in the medical field that only a judgment based on results obtained from actual administrations of the drug to patients does make sense. For example, it is a common sense in the global medical field that validity of recent medical judgments is based on Evidence-based medicine (EBM). In EBM, only results obtained by the actual administration of drugs to humans are adopted. If the medical practice is judged based solely on information about an enzyme in a patient and concentration of a substance, it is thought to be a problem. Therefore, even though there is a difference in an enzyme activity of MTHFR depending on A1298C polymorphism, a presumption that the fact has a direct relationship with an effective dose of the methotrexate may not be a valid scientific presumption.

"The method of examining which one of three types, A/A, A/C, or C/C A1298C polymorphism in the MTHFR gene belongs to" according to the present invention includes any methods by which an A1298C genotype present in the seventh exon in the MTHFR gene in an individual patient can be accurately examined. For example, the genotype can also be examined by using a DNA sequencer or a polymerase-chain reaction-restriction fragment length polymorphism (PCR-RFLP) method.
When the PCR-RFLP method is used for the examination, the method can be carried out as follows. First, DNA obtained from an individual patient and two synthetic DNAs each having the nucleotide sequence shown in SEQ ID NO: 1 and the nucleotide sequence shown in SEQ ID NO: 2 in Sequence Listing are mixed. A solution containing a necessary compound is added to the mixture to amplify the gene region including the A1298C polymorphism in the MTHFR gene by a polymerase-chain reaction (PCR). Further, the amplified DNA is cut by the restriction enzyme Fnu4I and the DNA is subjected to electrophoresis to examine whether the DNA is cut. As a result, it can be known which genotype, A/A, A/C, or C/C, the DNA has.

"Method for treatment of rheumatoid arthritis for determining a dose of methotrexate for each individual" in the present invention means a method in which the dose of the methotrexate for each patient is determined by using the test method of the present invention and the like as a laboratory test, the dose of the methotrexate is determined at start of the administration in each treatment, and the determination is made on how much the dose for each patient should be increased in the case of an insufficient effect.

Hereinafter, the present invention will be described in more detail by way of Example, but the present invention is not limited to the Example.

### Examples

### <Subject>

Target patients were 157 patients (M/F = 24/133) suffering from rheumatoid arthritis who were attending to Institute of Rheumatology Tokyo Women's Medical University Hospital. All of those patients were different from patients examined in Non-Patent Document 3.
Examinations were performed on genotypes of A1298C in the MTHFR gene in these 157 patients suffering from rheumatoid arthritis and doses of methotrexate to the patients 12 months after a start of administration.

### <Genomic DNA amplification reaction>

Genomic DNA was extracted from leukocytes in peripheral blood of each patient suffering from rheumatoid arthritis. From the genomic DNA, a DNA region including A1298C in the MTHFR gene was amplified by PCR. The primers shown in SEQ ID NO: 1 and SEQ ID NO: 2 in Sequence Listing were used for the amplification.
The DNA amplification reaction was performed in a system of a total volume of 50 µl, which contains 5 µl of 10xPCR Buffer (Applied Biosystem, Japan), 0.5 µl (= 2.5 U) of AmpliTaq Gold (Applied Biosystem, Japan), 5.0 µl of 8 mM dNTP (200 µM of each dNTP), and 1 µl of each 10 pmole primer (0.2 µM of each primer). Conditions of the PCR included: totally 34 cycles of denaturing at 95°C for 10 minutes, of reactions each at 95°C for 30 seconds, at 65°C for 30 seconds, and at 72°C for 60 seconds; and final extension at 72°C for 7 minutes.

### <Determination of genotype>

The products amplified by the PCR were cut by the restriction enzyme Fnu4I and subjected to electrophoresis to examine whether they were cut or not, to thereby determine the genotypes (A/A, A/C, C/C) in each patient.

### <Statistical processing>

Alleles and genotypes in group were compared by using Fisher's exact test for statistic analysis and Chi-square test, and Excel was used as a statistical software.

### <Study on a relationship between a genotype of A1298C in the MTHFR gene and an effective dose of methotrexate>

Table 2 shows a relationship between the genotype and the effective dose of the methotrexate in all subjects of patients suffering from the rheumatoid arthritis. The number of persons in each cell in Table 2 is shown by an integer value.

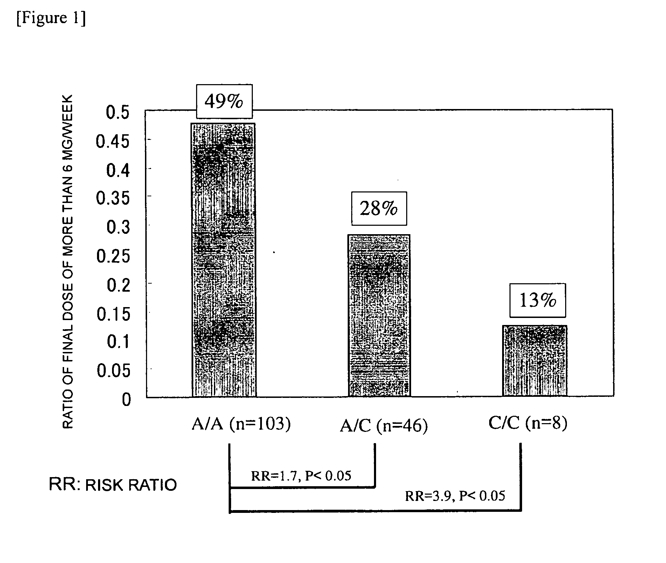

Table 2 shows a relationship between the genotype of A1298C polymorphism in the MTHFR and the dose of the methotrexate in all subjects of patients suffering from the rheumatoid arthritis. In the present Example, a dose of more than 6 mg/week 12 months after the start of the administration of the methotrexate was defined as a high dose, and otherwise defined as a low dose. Individuals were classified into A/A, A/C, and C/C based on the genotypes of A1298C polymorphism in the MTHFR and summarized to a relationship between the genotypes and the high dose. As shown in Fig. 1 and Table 2, a ratio of the high dose of the methotrexate was high in the order of A/A > A/C > C/C in the genotypes. In other words, it was indicated that individuals having the genotype including the A allele had a sharply-increased risk that the high dose is required compared with individuals having the C/C genotype (C allele only). A possibility that patients having the A/A genotype are required to have a high dose of the methotrexate is 49%. Compared with this, possibilities of patients having the A/C genotype and patients having the C/C genotype were 28% and 13%, respectively (Table 2 and Fig. 1). There were a difference of 1.7 (risk ratio, P<0.05) between A/A and A/C, and a difference of 3.8 (risk ratio, P<0.05) between A/A and C/C.

**[Table 2]**

| The relationship between A1298C and ratio of final dose of MTX in more than 6 mg/week 12 months after the start of administration (157 persons) | | | | |
|---|---|---|---|---|
| Genotype | A/A | A/C | C/C | Total |
| High dose | 50 | 13 | 1 | 64 |
| Low dose | 53 | 33 | 7 | 93 |
| Total | 103 | 46 | 8 | 157 |
| Ratio of high dose | 0.485*,** | 0.283* | 0.125** | 0.408 |

| | | | | |
|---|---|---|---|---|
| *1.72 (95% confidence interval: 1.04 to 2.84), P < 0.05 **3.88 (95% confidence interval: 0.61 to 24.6), P < 0.05 | | | | |

### [Comparative Example]

### <Distinction of the A/A genotype of A1298C in the MTHFR gene from other genotypes and study on a relationship between the A/A genotype and an effective dose of methotrexate and a relationship between the other genotypes and the effective dose of the methotrexate>

Table 3 shows a relationship between the A/A genotype of A 1298C and an effective dose of methotrexate and a relationship between the other genotypes (A/C and C/C) and the effective dose of the methotrexate in all the subjects of the patients suffering from rheumatoid arthritis. The number of persons in each cell of Table 3 is shown by an integer value.

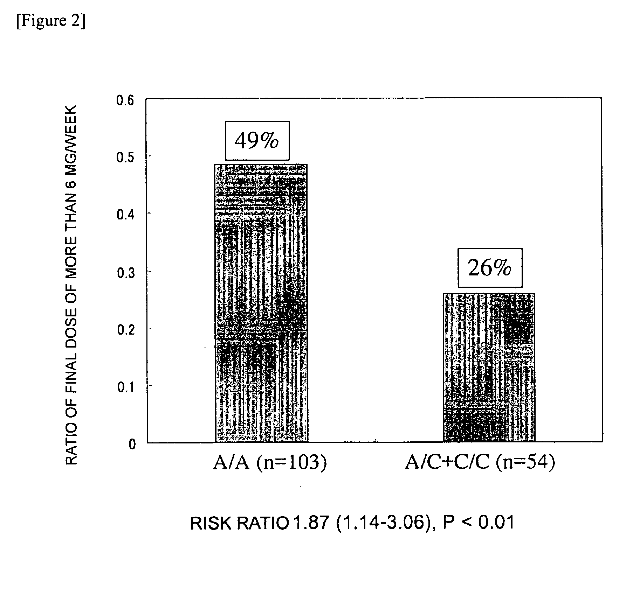

Table 3 and Fig. 2 show a relationship between the A/A genotype of A1298C polymorphism in the MTHFR and a dose and a relationship between the other genotypes (A/C and C/C) and a dose in all the subjects of the patients suffering from the rheumatoid arthritis. In the present reference example, the dose of more than 6 mg/week 12 months after the start of the administration of the methotrexate was defined as a high dose, and otherwise defined as a low dose. Genotypes of A1298C polymorphism in the MTHFR were classified into A/A and other genotype and summarized to a relationship between the genotypes and the high dose.
As shown in Fig. 2, 49% of individuals each having the A/A genotype of A1298C were classified to a high dose. Compared with this, 26% of individuals each having either A/C genotype or C/C genotype were classified to a high dose. In other words, a ratio of the individuals having the A/A genotype to whom more than 6 mg/week of the methotrexate were administered was 1.87 times higher than a ratio of the individuals having any of other genotypes. If the dose of more than 6 mg/week is considered to be risky, a risk ratio was 1.87 (95% confidence interval, 1.14 to 3.06), and Pearson's chi-square test to evaluate independence showed P < 0.01. The results were almost same as that of Table 5 in the Document 3.

**[Table 3]**

| Relationship between genotypes of A1298C polymorphism in MTHFR and a high dose | | | |
|---|---|---|---|
| Genotype | A/A | A/C + C/C | Total |
| High dose | 50 | 14 | 64 |
| Low dose | 53 | 40 | 93 |
| Total | 103 | 54 | 157 |
| Ratio of high dose | 0.485* | 0.259} | 0.408 |

| | | | |
|---|---|---|---|
| *Risk ratio 1.87 (95% confidence interval, 1.14 to 3.06) | | | |

By comparing Fig. 1 and Fig. 2, it was indicated that probabilities that an individual having the A/A genotype was administered in a high dose were the same (49%), but a probability that an individual having the A/C genotype was administered in the high dose increased from 26% to 28%, and a probability that an individual having the C/C genotype was administered in the high dose dropped from 26% to 13%. In other words, it was shown that the probability that the individuals having the C/C genotype needed the high dose decreased to about half. Therefore, it was first confirmed by the present invention that the probability of being low effective dose of methotrexate in the individuals having the C/C genotype (C allele only) is higher than the A/C genotype. The above results indicated that the examination method of the present invention may provide a genetic test method by which the probability for each individual may be accurately determined.

### [Industrial applicability]

By using the test method and method for treatment of the present invention, the dose in treatment at the start of the administration can be determined for each patient, and the determination on how much the dose for the each patient should be increased in the case of the insufficient effect of the drug can be made. Those methods can prevent the excessive administration of the methotrexate and enables the reduction of the burden on the patient body and the reduction of the medical expenses.

## Claims

1. A test method of determining an effective dose of methotrexate in a patient suffering from rheumatoid arthritis, comprising examining which one of three types, A/A, A/C, or C/C, an A1298C polymorphism in a methylenetetrahydrofolate reductase gene, MTHFR gene, in the patient belongs to.

2. A method of determining an effective dose of methotrexate for each individual patient by examining which one of three types, A/A, A/C, or C/C, an A1298C polymorphism in a methylenetetrahydrofolate reductase gene, MTHFR gene, in the patient belongs to.

3. A method according to Claim 2, comprising judging that a possibility of being high effective dose, is low in a patient having the C/C genotype (C allele only), medium in a patient having the A/C genotype, and high in a patient having the A/A genotype (A allele only).

4. A method for treatment of rheumatoid arthritis, comprising determining a dose of methotrexate for each individual using the method according to Claim 3 or 4.
